**Europäisches Patentamt**

**European Patent Office**

**Office européen des brevets**

(19)

(11) Veröffentlichungsnummer: **0 369 029**

**A1**

(12) # EUROPÄISCHE PATENTANMELDUNG
## veröffentlicht nach Art. 158 Abs. 3 EPÜ

(21) Anmeldenummer: **89905476.1**

(22) Anmeldetag: **25.04.89**

(86) Internationale Anmeldenummer:
**PCT/SU89/00111**

(87) Internationale Veröffentlichungsnummer:
**WO 89/10408 (02.11.89 89/26)**

(51) Int. Cl.⁵: **C12P 13/06, //C12N15/01, (C12P13/06,C12R1:13), (C12P13/06,C12R1:15)**

(30) Priorität: **26.04.88 SU 4408234**

(43) Veröffentlichungstag der Anmeldung:
**23.05.90 Patentblatt 90/21**

(84) Benannte Vertragsstaaten:
**BE DE FR GB IT SE**

(71) Anmelder: **NAUCHNO-ISSLEDOVATELSKY TEKHNOLOGICHESKY INSTITUT AMINOKISLOT NAUCHNO-PROIZVODSTVENNOGO OBIEDINENIA " ARMBIOTEKNOLOGIA "**
**ul. Gjurdzhiana 14**
**Erevan, 375056(SU)**

(72) Erfinder: **AZIZIAN, Asmik Gevorkovna**
**ul. Estonakan, 3-33**
**Erevan, 375038(SU)**
Erfinder: **AMBARTSUMIAN, Artur Albertovich**
**6-ya ul., 3 Noragavit**
**Erevan, 375000(SU)**
Erfinder: **KOCHARIAN, Shavarsh Mikaelovich**
**ul. Paroniana, 11-4**
**Erevan, 375015(SU)**
Erfinder: **ANANIKIAN, Maritsa Ananikovna**
**ul. Tikhogo Dona, 27-168**
**Erevan, 375087(SU)**

(74) Vertreter: **von Füner, Alexander, Dr. et al**
**Patentanwälte v. Füner, Ebbinghaus, Finck**
**Mariahilfplatz 2 & 3 Postfach 95 01 60**
**D-8000 München 95(DE)**

(54) VERFAHREN ZUR HERSTELLUNG VON L-ALANIN.

(57) Verfahren zur Herstellung von L-Alanin sieht die Kultivierung der Mutanten der Mikroorganismen der Arten Brevibacterium bzw. Corynebacterium, die für ihr Wachstum D-Alanin brauchen, an einem Nährboden, der assimilierbare Kohlenstoff- und Stickstoffquellen, anorganische Salze und Wuchsstoffe der Mikroorganismen enthält, bis zur Speicherung von L-Alanin in einer Kulturflüssigkeit und die darauffolgende Ausscheidung des Endproduktes vor.

EPAA-38600.5

## VERFAHREN ZUR HERSTELLUNG VON L-ALANIN

### Technisches Gebiet

Die Erfindung bezieht sich auf die mikrobiologische Industrie und insbesondere auf Verfahren zur Herstellung von Aminosäuren und noch genauer auf ein Verfahren zur Herstellung von L-Alanin durch Fermentierung.

### Zugrundeliegender Stand der Technik

Bekannt sind Verfahren zur Herstellung von L-Alanin durch Transformation aus der Asparaginsäure unter Zuhilfenahme von immobilisierunden Zellen der Mikroorganismen, die eine $\beta$-Dekarboxylase-Aktivität (US, A, 3899128) besitzen.

Solche bekannten Verfahren sind mehrstufig, was auf die Notwendigkeit zurückzuführen ist, in der ersten Stufe des Verfahrens, beispielsweise, die L-Asparaginsäure aus Ammoniumfumarat durch Transformation mit Hilfe von Aspartase zu gewinnen, und in der zweiten Stufe des Verfahrens die hergestellte L-Asparaginsäure mit Hilfe von L-Aspartat--$\beta$-dekarboxylase das L-Alanin zu tranformieren. (Progress in Industrial Microbiology, v. 24, 1986, Tokyo, J.Chibata, T.Tosa, T.Kakimoto "Alamine", p. 224-232).

Bekannt ist ein Verfahren zur Ausscheidung von L-Alanin aus einem razemischen Gemisch, bei dem man das D,L-Alanin der Azetylierung aussetzt und dann man unter Zuhilfenahme eines Fermenten der Aminoazylase das Azetyl--L-Alanin selektiv hydralysiert, wobei man das L-Alanin erhält. Danach erfolgt die Absonderung des L-Alanins vom Azetyl-D-alanin (US, A, 3386888).

Das genannte Verfahren ist mehrstufig und ist mit der Norwendigkeit verbunden, intermediäre D,L-Alanin, Azetyl-D,L-alanin und das Ferment der Aminoazylase zu gewinnen, was die Prozeßführung verkompliziert und verteuert.

### Offenbarung der Erfindung

Der vorliegenden Erfindung liegt die Aufgabe zugrunde, unter Verwendung eines neuen Typs von Mutanten der Mikroorganismen ein Verfahren zur Herstellung von L-Alanin zu entwickeln, welches es ermöglicht, die Prozeßführung der Gewinnung des Fertigproduktes mit Erreichung einer industriemäßig akzeptablen Ausbeute an Endprodukt in einer Kulturflüssigkeit zu vereinfachen.

Diese Aufgabe wurde dadurch gelöst, daß man im Verfahren zur Herstellung von L-Alanin, das durch Kultivierung von Mikroorganismen der Arten Brevibacterium und Corynebacterium am einem Nährboden, der assimilierbare Kohlenstoff- und Stickstoffquellen, anorganische Salze und organische Verbindungen enthält, die das Wachstum der Mikroorganismen stimulieren, bis zur Speicherung von L-Alanin in der Kulturflüssigkeit und durch , darauffolgende Ausscheidung des Endproduktes erfolgt, erfindungsgemäß, als Mikroorganismen die Mutanten der Mikroorganismen der Arten Brevibacterium und Corynebacterium verwendet, die für ihr Wachstum D-Alanin brauchen.

Durch die angemeldete Erfindung wurde es möglich, ein optisch reines L-Alanin in der Kulturflüssigkeit zu gewinnen, wobei die früher erforderlichen, mit der Ausscheidung des L-Alanins aus dem razemischen Gemisch verbundenen Stufen ausgeschlossen werden, dabei erreicht der Gehalt an Endprodukt in der Kulturflüssigkeit 28 g/l.

In Übereinstimmung mit der beanspruchfen Erfindung ist es zweckmäßig, die Mutanten der Mikroorganismen der Arten Brevibacterium flavum zu verwenden, die im Allunionsforschungsinstitut für Genetik und Selektion industriemäßiger Mikroorganismen (VNIIgenetika) - Vsesouzny mauchno-issledovatelsky institut genetiki i selektsii promyshlennykh mikroorganismov (VNII Genetika), UdSSR, 113545, Moskau, Dorozhny proezd, 1 - deponiert sind: Stamm Brevibacterium flavum AAI, registriert unter Nr. B-3061, am 5. April 1984; Stamm Brevibacterium flavum AA2, registriert unter Nr. B-3062 am 05. April 1984.

Für die spätere Vergrößerung des Gehalts an Endprodukt in einer Kulturflüssigkeit (der 44 g/l erreicht) ist es, erfindungsgemäß, zweckmäßig, Mutanten der Mikroorganismen der Art Brevibacterium zu verwenden, die gegenüber D,L-$\alpha$- Aminobuttersäure resistent sind.

In Übereinstimmung mit der vorliegenden Erfindung ist es zweckmäßig, die Mutanten der Mikroorganismen der Art Brevibacterium flavum zu verwenden, die in dem Allunionsforschungsinstitut für Genetik und Selektion der industriemäßigen Mikroorganismen (VNIIGenetik) deponiert sind:

Stamm Brevibacterium flavum AA5, registriert unter Nr. B-3991 am 01. April 1987,

Stamm Brevibacterium flavum AA6, registriert unter Nr. B-3992 am 01. April 1987.

Es ist außerdem zweckmäßig, in Übereinstimmung mit der angemeldeten Erfindung die Mutanten der Mikroorganismen der Art Corynebacterium glutaminicum zu verwenden, die in Allunionsforschungsinstitut für Genetik und Selektion der industriemäßigen Mikroorganismen (VNNIGenetik) deponiert sind:

Stamm Corynebacterium glutamicum AA41, registriert unter Nr. B-3321 am 25. März 1985,

Stamm Corynebacterium glutamicum AA11, registriert unter Nr. B-3323 am 25. März 1985.

Weitere Ziele und Vorzüge der angemeldeten Erfindung werden aus der nachstehenden ausführlichen Beschreibung des Verfahrens zur Herstellung von L-Alanin und aus den konkreten Beispielen der Realisierung des Verfahren ersichtlich.

Beste Ausführungsvariante der Erfindung

Das in der angemeldeten Erfindung vorgeschlagene Verfahren zur Herstellung von L-Alanin beruht auf der Verwendung von Mikroorganismen, Produzenten von L-Alanin, die in einem Nährboden kultiviert werden.

Es wird die Verwendung der Mutanten der Mikroorganismen der Arten Brevibacterium und Corynebacterium vorgeshclagen, die für ihr Wachstum D-Alanin brauchen. Als konkretes Beispiel für solche Mikroorganismen gilt ein Stamm, Produzent von L-Alanin, Brevibacterium flavum AA1, deponiert im Allunionsforschungsinstitut für Genetik und Selektion von industriemäßigen Mikroorganismen (VNIIGenetika) und registriert unter Nr. B-3061 am 05. April 1984;

ein Stamm, Produzent von L-Alanin, Brevibacterium flavum AA2, deponiert im Allunionsforschungsinstitut für Genetik und Selektion von industriemäßigen Mikroorganismen (VNIIGenetika) und registriert unter Nr. B-3062 am 05. April 1984;

ein Stamm- Produzent von L-Alanin, Corynebacterium glutamicum AA41, deponiert im Allunionsforschungsinstitut für Genetik und Selektion von industriemäßigen Mikroorganismen (VNIIGenetiks) und registriert unter Nr. B-3321 am 25. März 1985,

ein Stamm, Produzent von L-Alanin, Corynebacterium glutamicum AA11, deponiert im Allunionsforschungsinstitut für Genetik und Selektion von industriemäßigen Mikroorganismen (VNIIGenetika) und registriert unter Nr. B-3323 am 25. März 1985.

Erfindungsgemäß wird auch die Verwendung der Mutanten der Mikroorganismen der Art Brevibacterium vorgeschlagen, die D-Alanin brauchen und gegenüber D,L-$\alpha$-Aminobuttersäure resistent sind. Als Beispiele für solche Mikroorganismen können dienen

ein Stamm, Produzent von L-Alanin, Brevibacterium flavum AA5 oder Brevibacterium flavum AA6, deponiert im Allunionsforschungsinstitut für Genetik und Selektion von industriemäßigen Mikroorganismen (VNIIGenetika) und registriert unter Nr. B-3991 und B-3992 am 01. April 1987.

Die Mutationen der Auxotrophie nach D-Alanin können durch Behandlung der Mikroorganismen in einem beliebigen bekannten Verfahren (beispielsweise durch Behandlung mit N-Methyl-N'-nitro-N-nitrosoguanidin beziehungsweise mit UV-Strahlung) induziert. Die Mutationen der Resistenz gegenüber D,L-$\alpha$-Aminobuttersäure können entweder spontan sein, oder durch Behandlung der Mikroorganismen im beliebigen bekannten Verfahren induziert werden. Die Vereinigung in einem Genom der Mikroorganismen der Mutation der Resistenz geneüber der D,L-$\alpha$-Aminobutter-

säure und der Mutation der Auxotrophie nach D-Alanin kann in beliebiger Sequenz erfolgen.

Als Elternstämme für die Selektion von Mutanten, die D-Alanin brauchen, können beliebige Natur-Mikroorganismen oder Mutanten der Mikroorganismen verwendet werden, die Alanin im Form eines razemischen Gmmisches produzieren. Die obengenannten neuen Stämme Brevibacterium flavum AA1, Brevibacterium flavum AA2, Brevibacterium flavum AA6 und Brevibacterium flavum AA5 wurden mit genetischen Selektionstechniken aus dem Stamm Brevibacterium flavum ATCC 14067 gewonnen, der im Allunionsforschungsinstitut für Genetik und Selektion von industriemäßigen Mikroorganismen (VNIIGenetika) deponiert und unter Nr. B-42 am 16. Januar 1969 geristriert wurde. Die obengenannten neuen Stämme, Corynebacterium glutamicum AA41 und Corynebacterium glutaminicum AA11 wurden mit genetischen Selektionstechniken aus dem umfassend bekannten Stamm, Corynebacterium glutamicum ATCC 13032 gewonnen, der im Allunionsforschungsinstitut für Genetik und Selektion von industriemäßigen Mikroorganismen (VNIIGenetika) deponiert und unter Nr. B-41 am 16. Januar 1969 registriert wurde.

Als Elternstämme für die Selektion der in der angemeldeten Erfindung genannten Mikroorganismen beziehungsweise des vorgeschlagenen Typs von Mutanten, die D-Alanin brauchen, und die möglicherweise gegenüber der D,L-$\alpha$--Amunobuttersäure resistent sind, können jedoch beliebige Stämme der Mikroorganismen verwendet werden, die zur Art Brevibacterium oder zur Art Corynebacterium gehören.

Die Stämme AA1, AA2, AA41 und AA11 unterscheiden sich in ihren Kenndaten (außer der Auxotrophie nach D-Alanin und der L-alaninproduzierenden Fähigkeit) nicht von den Elternstämmen Brevibacterium flavum ATCC 14067 bzw. Corynebacterium glutamicum ATCC 13032.

Die vorgeschlagenen Stämme, Produzente von L-Alanin, Brevibacterium flavum AA1 und Brevibacterium flavum AA2 weisen folgende morphologisch-kulturelle und physiologisch-biochemische Merkmale auf.

Zellen sind oval, leicht ausgezogen, unbeweglich, nichtsporenbildend, grammpositiv. Die Kolonien am Fleich-Pepton-Agar sind nach 48 Stunden der Inkubation rund und weisen eine glatte, seltener eine rauhe Oberfläche mit gelber Färbung auf und erreichen einen Durchmesser von 2 mm.

Fakultative Anaerobier

Sie verflüssigen die Gelatine nicht. Sie vergären Glukose und Saccharose. Sie assimilieren Ammoniumstickstoff, Wachstumsoptimum liegt bei einer Temperatur von 30 bis 32°C und bei einem pH-Wert von 7,2 bis 7,8. Zum Wachstum an minimalen Nährböden brauchen sie auch Biotin und Thiamin.

Die Herstellung des Stammes Brevibacterium flavum AA1 und des Stammes Brevibacterium flavum AA2 wird wie folgt durchgeführt:

Den Stamm ATCC 14067 kultiviert man im Fleisch-Pepton-Bouillon bei einer Temperatur von 30°C unter Aeration bis zur Erreichung eines Titers von $10^9$ Zellen/ml. Die Zellen werden vom Fleisch-Pepton-Bouillon durch Zentrifugieren ausgewaschen und in einem Zitrat-Puffer (pH 5,5) resuspendiert. Der hergestellten Suspension setzt man N-Mehhyl-N'-nitro-N-nitrosoguanidin bis zur Erzielung einer Endkonzentration von 300 $\mu$g/ml zu, inkubiert man unter Aeration innerhalb von 20 Minuten bei einer Temperatur von 30°C. Dann werden die Zellen vom Mutagen mit gekühltem Fleisch-Pepton-Bouillon ausgewaschen, in Reagenzgläser mit einem Volumen von 10 ml Fleisch-Pepton-Bouillon übertragen, unter Aeration innerhalb von 18 Stunden bei einer Temperatur von 30° inkubiert und dann auf die Nährbodenoberfläche des Fleisch-Pepton-Bouillons, der 2 Gew,% Agar-Agar enthält, inokuliert. Die Kolonien, die an diesem Nährboden in 48 Stunden der Inkubation bei einer Temperatur von 30°C gewachsen, werden im Abdruckverfahren auf die Wachstumfähigkeit auf zwei Nährböden folgender Zusammensetzung geprüft: Nährboden 1: (mg/ml):

| | |
|---|---|
| Glukose | - 20,0 |
| $Na_2SO_4$ | - 2,0 |
| $K_2HPO_4$ | - 1,0 |
| $NH_4Cl$ | - 3,0 |
| $NH_4NO_3$ | - 1,0 |
| $MgSO_4 \cdot 7H_2O$ | - 0,1 |
| $MnSO_4 \cdot 5H_2O$ | - $1 \cdot 10^{-4}$ |
| $FeSO_4 \cdot 7H_2O$ | - $1 \cdot 10^{-4}$ |
| Biotin | - $5 \cdot 10^{-5}$ |
| Thiaminchlorid | - $1 \cdot 10^{-4}$ |
| Agar-Agar | - 20,0 |
| pH | - 7,4 . |

Die Zusammensetzung des Nährbodens 2 unterscheidet sich von der Zusammensetzung des Nährbodens 1 dadurch, daß er 100 $\mu$g/ml D-Alanin enthält. Die Mutanten Brevibacterium Flavum AA1 und Brevibacterium flavum AA2 wählt man aus den Kolonien aus, die zum Wachstum in 48 Stunden der Inkubation bei einer Temperatur von 30°C an dem Nährboden 1 nicht fähig sind und die an Nährboden 2 wachsen können.

Die vorgeschlagenen Stämme, Produzenten von L-Alanin, Corynebacterium glutaminicum AA41 und Corynebacterium glutaminicum AA11 weisen folgende morphologisch-kulturelle und physilogo-biochemische Merkmale auf.

Zellen sind oval, kurz und bilden keine Sporen.

Nach Gram färben sir sich positiv. Nach 48 Stunden der Inkubation an einem Fleich-Pepton-Agar sind sie rund, glänzend, cremefarben gefärbt; Kanten sind glatt, und sir erreichen einen Durchmesser von 2 bis 3 mm.

Fakultative Anaeribier. Sie lassen Milch nicht gerinnen. Verflüssigen Gelatine nicht. Vergären Glukose und Saccharose. Assimilieren Ammoniumstickstoff und Harnstoff. Das Wachstumsoptimum liegt bei einer Temperatur von 30 bis 32°C und einem pH-Wert von 7,2 bis 7,8.

Zum Wachstum an minimalen Nährböden brauchen sie Thiamin.

Die Stämme AA5 und AA6 unterscheiden sich in ihren Kenndaten (außer der Resistenz gegen D-,L-$\alpha$ Aminobutter-säure, der Auxotrophie nach D-Alanin und nach der L-alaninproduzierenden Fähigkeit) nicht von dem Elternstamm Brevibacterium Flavum ATCC 14067.

Die vorgeschlagenen Stämme, Produzenten von L-Alanin, Brevibacterium flavum AA5 und Brevibacterium flavum AA6 weisen folgende kultural-morphologische Eigenschaften auf:

Morphologische Merkmale.

Zellen sind oval, leicht ausgezogen, mit einer Länge von 1,7 bis 2,5 $\mu$m, sporenfrei, unbeweglich und gramm-positiv.

Kulturmerkmale.

Auf einem Fleisch-Pepton-Agar bilden am 2. Tag des Wachstums dei einer Temperatur von 30°C Kolonien mit einem Durchmesser von 2 mm, rund, glatt, gelblich gefärbt.

Strich am Fleisch-Pepton-Agar. Am 2. Tag ist das Wachstum mäßig, die Kanten sind glatt, die Oberfläche ist glänzend, dirht, die Farbe ist gelblich-cremefarben.

Einstich-Wachstum am Fleisch-Pepton-Agar ist mäßig, hauptsächlich an der Oberfläche des Nährbodens.

Gloverscher Mineralnährboden mit Glukose. Zum Wachstum sind Biotin, Thiamin und D-Alanin erforderlich. Am 2. Tag des Wachstum bei einer Temperatur von 30° bilden Kolonien mit einem Durchmesser von 1 mm, die Farbe ist hell-cremafarben. Das Wachstum des Striches am 2. Tag ist mäßig, dicht, die Farbe hellcremefarben.

Wachsen am Kartoffel und bilden ein Pigment mit gellicher Farbschattierung. Gelatine verflüssigen nicht.

Verhalten zu Kohlenstoffquellen. Vergären Glukose, Saccharose, Mannose, Fruktose und Maltose; schwächer vergären sie Galaktose, Rahmnose, Sorbose, Sorbit, Ammonium-und Natriumsalze der Essisäure, Äthylaloohol, sie vergären nicht Laktose.

Verhalten zu Stickstoffquellen. Sie assimilieren Ammoniumsulfat und Ammoniumchlorid sowie Harnstoff.

Wachsrumsoptimum liegt bei einer Temperatur von 30 bis 32°C und bei einem pH-Wert von 7,2 bis 7,8.

Züchtung des Stammes Brevibacterium flavum AA5 wird wie folgt durchgeführt.

Den Stamm Brevibacterium flavum ATCC 14067 züchtet man in einem Fleisch-Pepton-Bouillon bei einer Temperatur von 30°C mit Aeration bis zur Erreichung eines Titers von $10^9$ Zellen/ml. Die Zellen werden vom Fleisch--Pepton-Bouillon durch Zetrifugieren ausgewaschen und in einem Zitrat-Pufferlösung mit einem pH-Wert von 5,5 resuspendiert. Der hergestellten Suspension setzt man N-Methyl-N'-nitro-N-nitrosoguanidin bis zu einer Endkonzentration von 300 $\mu$g/ml zu und inkubiert man unter Aeration innerhalb von 20 Minuten bei einer Temperatur von 30°C. Dann werden die Zellen vom Mutagen mit abgekühlten Fleisch-Pepton-Bouillon ausgewaschen, in die Reagenzgläser mit 10 ml Fleisch-Pepton-Bouillon übertragen innerhalb von 18 Stunden bei einer Temperatur von 30°C inkubiert und dann auf die Oberfläche des Nährbodens Nr. 1 folgender Zusammensetzung (mg/ml) geimpft:

| | |
|---|---|
| Glukose | – 20,0 |
| $Na_2SO_4$ | – 2,0 |
| $K_2HPO_4$ | – 1,0 |
| $NH_4Cl$ | – 3,0 |
| $NH_4NO_3$ | – 1,0 |
| $MgSO_4 \cdot 7H_2O$ | – 0,1 |
| $MnSO_4 \cdot 5H_2O$ | – $1 \cdot 10^{-4}$ |
| $FeSO_4 \cdot 7H_2O$ | – $1 \cdot 10^{-4}$ |
| Biotin | – $5 \cdot 10^{-5}$ |
| Thiaminchlorid | – $1 \cdot 10^{-4}$ |
| Agar-Agar | – 20,0 |
| D,t-$\alpha$-Aminobuttersäure | – 30,0 |
| pH | – 7,4 . |

Die an diesem Nährboden Nr. 1 kultivierten Kolonien werden in 4 bis 5 Tagen der Inkubation bei einer Temperatur von 30°C gereinigt und auf ihre alaninproduzierende Fähigkeit überprüft. Eine der dadurch ausgewählten Mutanten, die die erhöhten Mengen von D,L-Alanin produziert, wird erneut der Mutagenese mit N-Methyl-N'-nitro-N-nitrosoguanidin ausgeseetzt, man nimmt Mutanten ab, die für ihr Wachstum D-Alanin brauchen. Der Stamm Brevibacterium flavum AA5, der L-Alanin produziert, stellt eine der auf diese Weise abgenommenen Mutanten dar.

Die Herstellung des Stammes Brevivacterium flavum AA6 wird die folgt durchgeführt.

Stamm Brevibacterium flavum ATCC 14067 mutagenisiert man mit N-Methyl-N'-nitro-N-nitrosoguynidin und wählt man eine Mutante aus, die D-Alanin braucht. Die gezüchtete Mutante, die für ihr Wachstum D-Alanin brauchen, werden erneut der Mutagenese mit N-Methyl-N'-nitro-N-nitrosoguanidin ausgesetzt, und man wählt die Mutanten aus, die egegen D,L-$\alpha$-Aminobuttersäure resistent sind, wie bei der Züchtung des Stammes Brevibacterium flavum AA5, in die Zusammensetzung des Nährbodens Nr. 1 führt man aber auch D-Alamin in einer Konzentration von 0,1 mg/ml ein. Eine der auf diese Weise gewählten Mutanten, die die erhöhten Mengen von L-Alanin produzieren, wird als Brevibacterium flavum AA6 bezeichnet.

Stämme Corynebacterium glutamicum AA11 und Corynebacterium glutamicum AA41 werden aus dem Stamm Corynebacterium glutamicum ATCC 13032 in Selektionstechniken wie bei dem obendenannten Techniken für die Züchtung der Stämme Brevibacterium flavum AA1 und Brevibacterium flavum AA2 aus dem Stamm Brevibacterium flavum ATCC 14067 gewonnen.

Die Unterscheidungsmerkmale der vorgeschlagenen Stämme im Vergleich zu den entsprechenden Elternstämmen Brevibacterium flavum ATCC 14067 und Corynebacterium glutamicum ATCC 13032 werden in der nachstehenden Tabelle angeführt:

| Stämme | Fähigkeit zum Wachstum an einem Nährboden, % | | | Fähigkeit zur Speicherung an einem Nährboden von D- und L-Alaninform, % | |
|---|---|---|---|---|---|
| | ohne D-Alanin | mit D-Alanin, 100 µg/ml | mit D-Alanin (100 µg/ml) D,L-$\alpha$-Aminobuttersäure (30 µg/ml) | D-Alanin | L-Alanin |
| Brevibacterium flavum ATCC 14067 | + | + | − | 45 | 55 |
| Brevibacterium flavum AA1 | − | + | − | 0 | 100 |
| Brevibacterium flavum AA2 | − | + | − | 0 | 100 |
| Brevibacterium flavum AA5 | − | + | + | 0 | 100 |
| Brevibacterium flavum AA6 | − | + | + | 0 | 100 |
| Corynebacterium glutamicum ATCC 13032 | + | + | − | 48 | 52 |
| Corynebacterium glutamicum AA11 | − | + | − | 0 | 100 |
| Corynebacterium glutamicum AA41 | − | + | − | 0 | 100 |

Zur Ermittlung des Bedarfs an D-Alanin für das Wachstum der in Tabelle genannten Stämme wird die Suspension dieser Stämme in Form einer Schleife auf die Oberfläche des Nährbodens Nr. 1 und Nr. 2 (die Zusammensetzung dieser Nährboden ist oben aufgeführt) aufgetragen und innerhalb von 40 Stunden bei einer Temperatur von 30°C inkubiert. Das Vorliegen von Wachstumsmerkmalen ist mit Zeichen "+" und das Fehlen derselben mit Zeichen "-" bezeichnet.

Die Stämme AA1, AA2, AA5, AA6, AA11, AA41 wachsen im Unterschied zu den Elternstämmen ATCC 14067 und ATCC 13032 am Nährboden Nr. 1 nicht.

Die Stämme AA5 und AA6 sind im Unterschied zu den übrigen Stämmen, die in der Tabelle angegeben sind, Fähig, an einem Nährboden zu wachsen, dessen Zusammensetzung der Zusammensetzung des Nährbodens Nr. 2 analog ist mit dem Unterschied aber dass in diesem auch 30 mg/ml D,L-$\alpha$-Aminobuttersäure entalten sind.

Zur Ermittlung der Fähigkeit der Stämme, D- und L-Stereoisomere des Alanins zu synthetisieren, werden die Suspensionen dieser Stämme in Reagenzgläser inokuliert, die 5 ml eines sterilisierten Nährbodens golgender Zusammensetzung enthalten: (g/l):

| | |
|---|---|
| Glukose | – 50% |
| $(NH_4)_2SO_4$ | – 30,0 |
| $KH_2PO_4$ | – 3,0 |
| $MgSO_4 \cdot 7H_2O$ | – 1,0 |
| D-Alanin | – 0,1 |
| $FeSO_4 \cdot 7H_2O$ | – 0,01 |
| $MnSO_4 \cdot 5H_2O$ | – 0,01 |
| Biotin | – 0,0002 |
| Thiaminbromid | – 0,00005 |
| $CaCO_3$ | – 30,0 , |

so berechnet, da die Konzentration der Mikroorganismen $10^7$ Zellen/ml beträgt. Die Reagenzgläser werden unter lleichtem Schukeln innerhalb von 40 Stunden bei einer Temperatur von 30°C inkubiert. In der hergestellten Kulturflüssigkeit ermittelt man den Gehalt an D,L-Alanin (Gesamtgehalt an D- und L-Formen von Alanin) im Papierchromatografie-Verfahren und durch Färbung mit Ninhydrin oder mittels des Aminosäurenanalysators. Der Gehalt an D-Alanin ermittelt man mit Hilfe eines Fermenten der D-Oxydase der Aminosäuren in einem Reaktionsgemisch bis auf ein Endvolumen von 0,4 ml, das enthält: 8 Millieinheiten der Oxydase der D-Aminosäuren aus Schweinennieren, 0,2 ml der zu prüfenden Lösung mit einem Gehalt an D-Alanin von 0,1 bis 5,0 mMol, 33 mMol eines Pyrophosphat--Puffers mit einem pH-wert von 8,3. Die Reaktion führt man bei einer Temperatur von 37°C bis zur Erreichung des Gleichgewichts, bei dem 93 bis 95% von D-Alanin zum Pyruvat umgesetzt werden. Das angefallene Pyruvat ermittelt man spektrofotometrisch bei einer Wellenlänge von 440 nm. Dann wird auf der Grundlage der gemessenen Werte des Gesamtgehaltes an D,L-Alanin und D-Alanin das Prozentverhältnis der D- und L-Formen von Alanin in Kulturflüssigkeiten berechnet.

Die erzielten Angaben sind in der obengenannten Tabelle angeführt. Aus der Tabelle geht hervor, da  die Stämme AA1, AA2, AA5, AA6, AA11, AA41 im Unterschied zu den Stämmen ATCC 14067 und ATCC 13032 nicht fähig sind, D-Alanin zu produzieren.

Das Inokulum der Produzentenstämme für die anschlie-ßende Fermentierung kann in beliebigem bekanntem Verfahren, solchem wie die Züchtung an der Oberfläche von Nährböden oder in flüssigen Nährböden zubereitet werden, die assimilierbare Kohlenstoff- und Stickstoffquelelen, anorganische Salze und organische Verbindungen enthalten, die das Wachstum der Mikroorganismen bewirken.

Zu den organischen Substanzen gehären Vitamine (Biotin, Thiamin) sowie andere Verbindungen, darunter Aminosäuren, in dem Fall, wenn sie für das Wachstum eines konkreten Produzentenstammes erforderlich waren.

Als Fermentation-Nährboden für die Kultivierung der Mutanten der Mikroorganismen, die in der vorliegenden Erfindung verwendet werden, können beliebige Nährböden eingesetzt werden, die assimilierbare Kohlenstoff- und Stickstoffquellen, anorganische Salze und organische Substanzen enthalten, die das Wachstum der Mikroorganismen und die Speicherung von L-Alanin stimulieren.

Als assimilierbare Kohlenstoffquellen können solche Kohlenstoffquellen wie Saccharose, Glukose, Fruktose, Maltose, Mannose, Stärke, Stärkehydrolysat und Melasse; mehrwertige Alkohole, solche wie Glyzerin und Sorbit; organische Säuren, solche wie Ameisesäure, Essigsäure, Milchsäure, Fumarsäure, Meleinsäure, Proplonsäure; solche Alkohole wie Methanol und Äthanol verwendet werden. Die genannten Kohlenstoffquellen können sowohl im Einzelnen als auch in Verbindung miteinander in unterschiedlichen Gewichtsverhältnissen berwendet werden. Die Gesamtmenge einer Substanz, Kohlenstoffquelle, kann zu Beginn der Fermentation oder portionsweise im Verlaufe der Fermentation eingeführt werden.

Als assimilierbare Stickstoffquelle können sowohl organische als auch anorganische Ammoniumsalze, beispielsweise Ammoniumsulfat, Ammoniumchlorid, Ammoniumkarbonat, Ammoniumazetat, Ammoniumnitrat, Ammoniumphosphat, Ammoniumlaktat und Ammonium; Harnstoff; unterschiedliche natürliche stickstoffhaltige Verbindungen, beispielsweise Pepton, Hefehydrolysat, Fleischextrakt und Hydrolysate von pflanzlichen Eiweißstoffen verwendet werden.

Als anorganische Salze können Kaliumphosphat, Natriumphosphat, Magnesiumsulfat, Natriumchrorid, Eisen-, Mangan- und Zinksalze sowie Kalziumkarbonat verwendet werden.

Als organische Stoffe, die für das Wachstum der obengenannten Mikroorganismen erforderlich sind, können Thiamin, Biotin beziehungsweise seine Substituenten sowie Aminosäuren verwendet werden, wenn soe für das Wachstum der Mikroorganismen erforderlich sind. Wenn organische Stoffe in einer ausreichenden Menge in der Zusammensetzung der anderen Komponenten eines Nährbodens vorhanden sind, entfällt die Notwendigkeit, organische Stoffe in reiner Form einzubringen.

Die Speicherung von L-Alanin kommt bei der Kultivierung unter anaeroben Bedingungen in einem Temperaturrenbereich von 20 bis 37°C und einem Bereich der pH-Werte von 6,5 bis 9,0 zustande.

Das Entstehen von L-Alanin wird in 6 bis 10 Stunden nach dem Beginn der Fermentation beobachtet. Das maximale Niveau der Speicherung von L-Alanin in einer Kulturflüssigkeit wird durch den vollständigen Vernrauch einer Kohlenstoffquelle und Energie erreicht (in 32 Stunden und darüber hinaus in Abhängigkeit von der verwendeten Konzentration einer Kohlenstoffquelle und Energie).

Den Gehalt an L-Alanin in einer Kulturflüssigkeit ermittelt man mit Hilfe der Papierchromatografie und der Färbung mit Ninhydrin beziehungsweise mit Hilfe eines Aminosäure-Analysators.

Das aufgespeicherte L-Alanin kann aus einer Kulturflüssigkeit in beliebigem bekanntem Verfahren, solchem wie die Beseitigung von Mikroorganismen und anderen unlöslichen Teilchen durch Zentrifugieren oder Filtration, durch Adsorption von L-Alanin an Ionenaustauscherharzen mit anschließendem Eluieren, Konzentrierung und Kristallisation von L-Alanin ausgeschieden werden.

Zur besseren Erläuterung der vorliegenden Erfindung werden nachstehende Beispiele für ihre konkrete Ausführung aufgeführt.

B e i s p i e l    1

Das Inokulumsmaterial des Stämmes Brevibacterium flavum AA2 stellt man durch Züchben der Mikroorganismen in den Reagenzgläsern an der Oberfläche der abgeschrägten Fleisch-Pepton-Bouillon, vor, die 15 g/l Agar-Agar und 100 ml/l D-Alanin enthält, innerhalb von 22 Stunden bei einer Temperatur von 30°C und darauffolgendes Abspüben der Mikroorganismen mit einer sterilen physiologischen Lösung (Konzentration der Mikroorganismen in der erhaltenen Suspension beträgt $10^9$ Zellen/mg) her.

In Fermentationkolben mit einem Volumen von 250 ml werden aseptisch je 10 ml sterilisierten Fermentationsnährboden folgender Zusammensetzung (g/l) vergossen: Melasse - 200,0 (nach Zucker - 100), Hefebiomasse-Hydrolysat - 120,0, $CaCO_3$ - 30,0, pH = 7,5.

In die Kolben bringt man je 0,5 $\mu$l Inokulumsmaterials des Stammes Brevibacterium flavum AA2 ein und inkubiert man unter leichtem Schaukeln (70 U/min) innerhalb von 72 Stunden bei einer Pemteratur von 30°C. Der Gehalt an L-Alanin in der Kulturflüssigkeit, die nach dem Abschluß der Fermentation anfällt, beträgt 5,1 g/l.

250 ml der erhaltenen Kulturflüssigkeit des Stammes Brevibacterium flavum AA2, die 5,1 g L-Alanin enthält, zentrifugiert man (5000 U/min innerhalb von 20 Minuten) zum Entfernen von Bakterien und anderen unlöslichenteichen. Der überstand wird über die Säule mit Ionenausfauscherharz in $NH_4^+$-Form in Richtung von oben nach unter. Bei einer Geschwindigkeit von 0,6 bis 0,7 Volumen der Lösung je ein Volumen des Harzes pro 1 Stunde geleitet. Die Säule wird mit Wasser gewaschen und das adsorbierte L-Alanin wird mit einer 3,5%igen wässerigen Ammoniaklösung eluiert. Das erhaltene Eluat konsentriert man unter-Vakuum und Kristallisiert man das L-Alanin bei einer Temperatur von 14 bis 16°C im Verlaufe von 4 Stunden. Kristalle werden von der Lösung durch Filterung durch Papierfilter abgetrennt und unter Vakuum getrocknet.

Die Ausbeute an L-Alanin beträgt 3,3 g ohne. Berücksicktigung des Gelaltes der Mutterlösung an L-Alanin, was eine Gesamtausbeute von 65%, bezogen auf den Gehalt in der Kulturflüssigkeit ergibt.

Der Reinheibsgrad des Produktes beträgt nach den Angaben der Papierchromatografie 77,4%.

B e i s p i e l    2

Das Inokulumsmaterial des Stammes Brevibacterium flavum AA2 stellt man unter den dem Beispiel 1 analogen Bedingungen her.

In Fermentationskolben mit einem Volumen von 250 ml vergießt man je 10 ml sterilisierten Fermentationsnährboden folgender Zusammensetzung (g/l): Melasse - 200,0 (nach Zucker -100); Hefebiomasse-Hydrolysat - 120,0; $CaCO_3$ - 30,0, pH = 7,5.

In die Kolben bringt man je 0,5 ml Inokulumsmaterial des Stammes Brevibacterium flavum AA1, der unter den in Beispiel 1 genannten Bedingungen gezüchtet wird, ein. Die Kolben werden unter leichtem Schaukeln (70 U/min) innerhalb von 72 Stunden bei einer Temperatur von 30°C inkubiert. Der Gehalt an L-Alanin in der Kulturflüssigkeit, die nach der Beendigung der Fermentation anfällt, beträgt 6,9 g/l.

B e i s p i e l   3

In Fermentationskolben mit einem Volumen von 250 ml vergießt man aseptisch je 10 ml sterilisierten Fermentationsnährboden folgender Zusammensetzung (g/l): Melasse - 200,0 (nach Zucker - 100); Hefebiomasse-Hydro-lysat - 120,0, $CaCO_3$ - 30,0, pH = 7,5.

In die Kolben bringt man je 0,5 ml Inokulumsmaterial des Stammes Corynebacterium glutamicum AA11, der unter den in Beispiel 1 genannten Bedingungen gezüchtet wird, ein. Die Kolben werden unter leichtem Schaukeln (70 U/min) innerhalb von 72 Stunden bei einer Temperatur von 30°C inkubiert. Der Gehalt an L-Alanin in der Kulturflüssigkeit, die nach der Beendigung der Fermentation anfällt, beträgt 3,8 g/l.

B e i s p i e l   4

. In Fermentationskolben mit einem Volumen von 250 ml vergießt man aseptisch je 10 ml sterilisierten Fermentationsnährboden folgender Zusammensetzung (g/l): Melasse - 200,0 (nach Zucker - 100), Hefebiomasse-Hydrolysat - 120,0, $CaCO_3$ - 30,0, pH = 7,5.

In die Kolben bringt man je 0,5 ml Inokulumsmaterial des Stammes Corynebacterium glutamicum AA41, der unter den in Beispiel 1 genannten Bedingungen gezüchtet wird, ein. Die Kolben werden unter leichtem Schaukeln (70 U/min) innerhalb von 72 Stunden bei einer Temperatur von 30°C inkubiert. Der Gehalt an L-Alanin in der Kulturflüssigkeit, die nach Beendigung der Fermentation anfällt, beträgt 6,4 g/l.

B e i s p i e l   5

Den Fermentationsproze führt man unter den in Beispiel 1 genannten Bedigungen durch, wobei man als Produzentenstammen von L-Alanin den Stamm Brevibacterium flavum AA1 benutzt man verwendet aber einen Fermentationsnährboden folgender Zusammensetzung (g/l):

| | |
|---|---|
| Saccharose | − 100,0 |
| $(NH_4)_2SO_4$ | − 55,0 |
| $KH_2PO_4$ | − 3,0 |
| $MgSO_4 \cdot 7H_2O$ | − 1,0 |
| D-Alanin | − 0,1 |
| $FeSO_4 \cdot 7H_2O$ | − 0,01 |
| $MnSO_4 \cdot 5H_2O$ | − 0,01 |
| Biotin | − 0,0002 |
| Thiaminbromid | − 0,00005 |
| $CaCO_3$ | − 50,0 |
| pH | − 7,5. |

Der Gehalt an L-Alanin in der Kulturflüssigkeit nach 72 Stunden Inkubation beträgt 17,6 g/l.

B e i s p i e l   6

Den Fermentationsproze führt man unter den in Beispiel 1 genannten Bedingungen durch, als Produzentenstamm von L-Alanin verwendet man aber den Stamm Brevibacterium flavum AA2 und man verwendet einen Fermentationsnährboden folgender Zusammensetzung (g/l):

| | |
|---|---|
| Saccharose | − 100,0 |
| $(NH_4)_2SO_4$ | − 55,0 |
| $KH_2PO_4$ | − 3,0 |
| $MgSO_4 \cdot 7H_2O$ | − 1,0 |
| D-Alanin | − 0,1 |
| $FeSO_4 \cdot 7H_2O$ | − 0,01 |
| $MnSO_4 \cdot 5H_2O$ | − 0,01 |

| | | |
|---|---|---|
| Biotin | – | 0,0002 |
| Thiaminbromid | – | 0,00005 |
| $CaCO_3$ | – | 50,0 |
| pH | = | 7,5 . |

Der Gehalt an L-Alanin in der Kulturflüssigkeit nach 72 Stunden der Inkubation beträgt 20,4 g/l.

B e i s p i e l 7

Den Fermentationsproze führt man unter den in Beispiel 1 genannten Bedingungen durch, man verwendet aber als Produzentenstamm von L-Alanin den Stamm Corynebacterium glutamicum AA11 und man verwendet einen Fermentationsnährboden folgender Zusammensetzung (g/l):

| | | |
|---|---|---|
| Saccharose | – | 100,0 |
| $(NH_4)_2SO_4$ | – | 55,0 |
| $KH_2PO_4$ | – | 3,0 |
| $MgSO_4 \cdot 7H_2O$ | – | 1,0 |
| D-Alanin | – | 0,1 |
| $FeSO_4 \cdot 7H_2O$ | – | 0,01 |
| $MnSO_4 \cdot 5H_2O$ | – | 0,01 |
| Biotin | – | 0,0002 |
| Thiaminbromid | – | 0,00005 |
| $CaCO_3$ | – | 50,0 |
| pH | = | 7,5 . |

Der Gehalt an L-Alanin in der Kulturflüssigkeit nach 72 Stunden der Inkubation beträgt 2,6 g/l.

B e i s p i e l 8

Der Fermentationsproze führt man unter den in Beispiel 1 genannten Bedingungen, man verwendet aber als Produzentenstamm von L-Alanin den Stamm Corynebacterium glutamicum AA41 und man verwendet einen Fermentationsnährboden folgender Zusammensetzung (g/l):

| | | |
|---|---|---|
| Saccharose | – | 100,0 |
| $(NH_4)_2SO_4$ | – | 55,0 |
| $KH_2PO_4$ | – | 3,0 |
| $MgSO_4 \cdot 7H_2O$ | – | 1,0 |
| D-Alanin | – | 0,1 |
| $FeSO_4 \cdot 7H_2O$ | – | 0,01 |

| | | |
|---|---|---|
| $MnSO_4 \cdot 5H_2O$ | – | 0,01 |
| Biotin | – | 0,00002 |
| Thiaminbromid | – | 0,00005 |
| $CaCO_3$ | – | 50,0 |
| pH | = | 7,5 . |

Der Gehalt an L-Alanin in der Kulturflüssigkeit nach 72 Stunden der Inkubation beträgt 5,1 g/l.

B e i s p i e l  9

Den Fermentationsproze führt man unter den in Beispiel 5 genannten Bedingungen durch, man bringt in die Kolben als Produzentenstamm von L-Alanin den Stamm Brevibacterium flavum AA5 ein. Der Fermentationsnährboden weist folgende Zusammensetzung (g/l) auf:

| | | |
|---|---|---|
| Saccharose | – | 120,0 |
| $(NH_4)_2SO_4$ | – | 55,0 |
| $KH_2PO_4$ | – | 3,0 |
| $MgSO_4 \cdot 7H_2O$ | – | 1,0 |
| D-Alanin | – | 0,1 |
| $FeSO_4 \cdot 7H_2O$ | – | 0,01 |
| $MnSO_4 \cdot 5H_2O$ | – | 0,01 |
| Biotin | – | 0,0002 |
| Thiaminbromid | – | 0,00005 |
| $CaCO_3$ | – | 50,0 |
| pH | = | 7,5 . |

Die Kolben inkubiert man unter leichtem Schaukeln innerhalb von 72 Stunden bei einer Temperatur von 30°C. Der Gehalt an L-Alanin in der Kulturflüssigkeit, die nach der Beendigung der Fermentation anfällt, beträgt 32,9 g/l.

B e i s p i e l  10

Den Fermentationsproze führt man unter den in Beispiel 5 genannten Bedingungen. In die Kolben bringt man las Produzentenstamm von L-Alanin den Stamm Brevibacterium flavum AA6. ein.

Der Fermentationsnährboden hat folgende Zusammensetzung (g/l):

| | | |
|---|---|---|
| Saccharose | - | 120,0 |
| $(NH_4)_2SO_4$ | - | 55,0 |
| $KH_2PO_4$ | - | 3,0 |
| $MgSO_4 \cdot 7H_2O$ | - | 1,0 |
| D-Alanin | - | 0,1 |
| $FeSO_4 \cdot 7H_2O$ | - | 0,01 |
| $MnSO_4 \cdot 5H_2O$ | - | 0,01 |
| Biotin | - | 0,0002 |
| Thiaminbromid | - | 0,00005 |
| $CaCO_3$ | - | 50,0 |
| pH | = | 7,5 . |

Die Kolben inkubiert man unter leichtem Schaukeln innerhalb von 72 Stunden bei einer Temperatur von 30°C. Der Gehalt an L-Alanin in der Kulturflüssigkeit, die nach Beedigung der Fermentation anfällt, beträgt 31,4 g/l.

B e i s p i e l   11

Den Fermentationsproze  führt man unter den in Beispiel 5 genannten Bedigungen durch. Man bringt in die Kolben als Produzentenstann von L-Alanin den Stamm Brevibacterium flavum AA1 ein. Der Fermentationsnährboden hat folgende Zusammensetzung (g/l):

| | | |
|---|---|---|
| Saccharose | - | 120,0 |
| $(NH_4)_2SO_4$ | - | 55,0 |
| $KH_2PO_4$ | - | 3,0 |
| $MgSO_4 \cdot 7H_2O$ | - | 1,0 |
| D-Alanin | - | 0,1 |
| $FeSO_4 \cdot 7H_2O$ | - | 0,01 |
| $MnSO_4 \cdot 5H_2O$ | - | 0,01 |
| Biotin | - | 0,0002 |
| Thiaminbromid | - | 0,00005 |
| $CaCO_3$ | - | 50,0 |
| pH | = | 7,5 . |

Die Kolben inkubiert man unter leichtem Schaukeln innerhalb von 72 Stunden bei einer Temperatur von 30°C. Der Gehalt an L-Alanin in der Kulturflüssigkeit, die nach Beendigung der Fermentation anfällt, beträgt 20,6 g/l.

B e i s p i e l 12

Den Fermentationsprozeß führt man unter den in Beispiel 5 genannten Bedingungen durch. In die Kolben bringt man jedoch je 0,5 ml Inokulumsmaterial des Stammes Brevibacterium flavum AA5 ein, der eine Suspension darstellt, die durch Abspülen von eintägigen agarisierten Abschrägungen des Fleisch-Pepton-Bouillons hergestellt wird (Konzentration an Mikroorganismen $10^9$ Zellen/ml). Der Fermentationsnährboden hat folgende Zusammensetzung (g/l):

| | |
|---|---|
| Saccharose | – 150,0 |
| $(NH_4)_2SO_4$ | – 55,0 |
| $KH_2PO_4$ | – 3,0 |
| $MgSO_4 \cdot 7H_2O$ | – 1,0 |
| D-Alanin | – 0,1 |
| $FeSO_4 \cdot 7H_2O$ | – 0,01 |
| $MnSO_4 \cdot 5H_2O$ | – 0,01 |
| Biotin | – 0,0002 |
| Thiaminbromid | – 0,00005 |
| $CaCO_3$ | – 50,0 |
| pH | = 7,5 . |

Die Kolben inkubiert man unter leichten Schaukeln innerhalb von 96 Stunden bei einer Temperatur von 30°C. Der Gehalt an L-Alanin in der Kulturflüssigkeit, die nach der Beendigung der Fermentation anfällt, beträgt 32,9 g/l.

Man führt die Reinigung von L-Alanin aus 250 ml Kulturflüssigkeit des Stammes Brevibacterium flavum AA5 (Gesamtgehalt an L-Alanin beträgt 11 g) unter den Bedingungen, die den in Beispiel 1 beschriebenen ähneln, durch. Die Ausbeute an L-Alanin ohne Berücksichtigung auf seinen Gehalt in Muterlange beträgt 7,3 g, was eine Gesamtausbeute, bezogen auf den Gehalt in der Kulturflüssigkeit, von 67% ergibt. Der Reinheitsgrad des Produktes beträgt nach den Angaben der Papierchromatografie 91,3%.

B e i s p i e l   13

Den Fermentationsproze führt man unter den in Beispiel 5 genannten Bedingungen durch. In die Kolben bringt man jedoch je 0,5 ml Inokulumsmetrial des Stammes Brevibacterium flavum AA6 ein, der eine Suspension darstellt, die durch Abspülen von eintägigen agarisierten Abschrägungen des Fleisch-Pepton-Bouillons hergestellt wird (Konzentration an Mikroorganismen $10^9$ Zellen/ml). Der Fermentationsnährboden hat folgende Zusammensetzung (g/l):

| | |
|---|---|
| Saccharose | - 150,0 |
| $(NH_4)_2SO_4$ | - 55,0 |
| $KH_2PO_4$ | - 3,0 |
| $MgSO_4 \cdot 7H_2O$ | - 1,0 |
| D-Alanin | - 0,1 |
| $FeSO_4 \cdot 7H_2O$ | - 0,01 |
| $MnSO_4 \cdot 5H_2O$ | - 0,001 |
| Biotin | - 0,0002 |
| Thiaminbromid | - 0,00005 |
| $CaCO_3$ | - 50,0 |
| pH | = 7,5 . |

Die Kolben werden unter leichtem Schaukeln innerhalb von 96 Stunden bei einer Temperatur von 30°C inkubiert. Der Gehalt an L-Alanin in der Kulturflüssigkeit, die nach der Beendigung der Fermentation anfällt, beträgt 42,3 g/l.

B e i s p i e l   14

Den Fermentationsprozeß führt man unter den in Beispiel 5 genannten Bedingungen durch. In die Kolben bringt man jedoch je 0,5 ml Inokulumsmaterial des Stammes Brevibacterium flavum AA1 ein, der eine Suspension darstellt, die durch Abspülen von eintägigen agarisierten Abschrägungen der Fleisch-Pepton-Bouillon hergestellt wird (Konzentration an Mikroorganismen $10^9$ Zellen/ml). Der Fermentationsnährboden hat folgende Zusammensetzung (g/l):

GEÄNDERTE PATENTANSPRÜCHE ZUR INTERNATIONALEN
ANMELDUNG PCT/SU 89/00111

1. Verfahren zur Herstellung von L-Alanin durch Kultivierung von Mikrooganismen der Arten Brevibacterium und Corynebacterium an einem Nährboden, der assimilierbare Kohlenstoff- und Stickstoffquellen, anorganische Salze und Wuchsstoffe der Mikrooganismen enthält, bis zur Speicherung von L-Alanin in einer Kulturflüssigkeit, und durch darauffolgende Ausscheidung des Enproduktes, dadurch g e k e n n z e i c h n e t , daß man als Mikroorganismen Mutanten der Mikroorganismen der Arten Brevibacterium bzw. Corynebacterium verwendet, die für ihr Wachstum D-Alanin brauchen.

2. Verfahren nach Anpsuuch 1, dadurch g e k e n n z e i c h n e t , da man die Mutanten der Mikroorganismen der Art Brevibacterium flavum verwendet, die im Allunionsforschunginstitut für Genetik und Selektion von industriemä igen Mikroorganismen (VNIIGenetika) deponiert sind:

Stamm Brevibacterium flavum AA1, registriert unter Nr. B-3061 am 05. April 1984,

Stamm Brevibacterium flavum AA2, registriert unter Nr. B-3062 am 05. April 1984.

3. (geändert) Verfahren nach Anspruch 1, dadurch g e k e n n z e i c h n e t , daß man die Mutanten der Mikroorganismen der Art Brevibacterium verwendet, die für ihr Wachstum D-Alanin brauchen und gegen D,L-$\alpha$-Aminobuttersäure resistent sind.

4. Verfahren nach Anspruch 1,3, dadurch g e k e n n z e i c h n e t , daß man die Mutanten der Mikroorganismen der Art Brevibacterium flavum verwendet, die im Allunionsforschungsinstitut für Genetik und Selektion von industriemäßigen Mikroorganismen (VNIIGenetika) deponiert sind:

Stamm Brevibacterium flavum AA5, registriert unter Nr. B-3991 am 01. April 1987,

5. Verfahren nach Anspruch 1, dadurch g e k e n n - z e i c h n e t , daß man die Mutanten der Mikroorganismen der Art Corynebacterium glutamicum verwendet, die im Allunionsforschungsinstitut für Genetik und Selektion von industriemäßigen Mikroorganismen (VNIIGenetika) deponiert sind:

Stamm Corynebacterium glutamicum AA41, registriert unter Nr. B-3321 am 25. März 1985,

Stamm Corynebacterium glutamicum AA11, registriert unter Nr. B-3323 am 25. März 1985.

6. Biologisch reine Kulturen der Mikroorganismen, die Kennlinien aufweisen und mit einem beliebigen der nachstehend aufgeführten Stämme-Produzenten von L-Alanin, identifiziert werden:

Brevibacterium flavum AA1;

Brevibacterium flavum AA2;

Brevibacterium flavum AA5;

Brevibacterium flavum AA6;

Corynebacterium glutamicum AA41;

Corynebacterium glutamicum AA11.

# PATENTANSPRÜCHE

1. Verfahren zur Herstellung von L-Alanin durch Kultivierung von Mikroorganismen der Arten Brevibacterium und Corynebacterium an einem Nährboden, der assimilierbare Kohlenstoff- und Stickstoffquellen, anorganische Salze und Wuchsstoffe der Mikroorganismen enthält, bis zur Speicherung von L-Alanin in einer Kulturflüssigkeit, und durch darauffolgende Ausscheidung des Endproduktes, dadurch g e k e n n z e i c h n e t , daß man als Mikroorganismen die Mutanten der Mikroorganismen der Arten Brevibacterium bzw. Corynebacterium verwendet, die für ihr Wachstum D-Alanin brauchen.

2. Verfahren nach Anspruch 1, dadurch g e k e n n - z e i c h n e t , daß man die Mutanten der Mikroorganismen der Art Brevibacterium flavum verwendet, die im Allunionsforschungsinstitut für Genetik und Selektion von industriemä igen Mikroorganismen (VNIIGenetika) deponiert sind:

Stamm Brevibacterium flavum AA1, registriert unter Nr. B-3061 am 05. April 1984,

Stamm Brevibacterium flavum AA2, registriert unter Nr. B-3062 am 05. April 1984.

3. Verfahren nach Anspruch 1, dadurch g e k e n n - z e i c h n e t , da man die Mutanten der Mikroorganismen der Art Brevibacterium verwendet, die gegen D,L-$\alpha$-
-Aminobuttersäure resistent sind.

4. Verfahren nach Anspruch 1,3, dadurch g e - k e n n z e i c h n e t , daß man die Mutanten der Mikroorganismen der Art Brevibacterium flavum verwendet, die im Allunionsforschungsinstitut für Genetik und Selektion von industriemäßigen Mikroorganismen (VNIIGenetika) deponiert sind:

Stamm Brevibacterium flavum AA5, registriert unter Nr. B-3991 am 01. April 1987,

Stamm Brevibacterium flavum AA6, registriert unter Nr. B-3992 am 01. April 1987.

| Saccharose | – | 150,0 |
| $(NH_4)_2SO_4$ | – | 55,0 |
| $KH_2PO_4$ | – | 3,0 |
| $MgSO_4 \cdot 7H_2O$ | – | 1,0 |
| D-Alanin | – | 0,1 |
| $FeSO_4 \cdot 7H_2O$ | – | 0,001 |
| $MnSO_4 \cdot 5H_2O$ | – | 0,01 |
| Biotin | – | 0,0002 |
| Thiaminbromid | – | 0,00005 |
| $CaCO_3$ | – | 50,0 |
| pH | = | 7,5 . |

Die Kolben werden unter leichtem Schaukeln innerhalb von 96 Stunden bei einer Temperatur von 30°C inkubiert. Der Gehalt an L-Alanin in der Kulturflüssigkeit, die nach der Beendigung der Fermentation anfällt, beträgt 28,1 g/l.

Gewerbliche Verwertbarkeit

Die Angemeldete Erfindung wird in der Medizin, in der chemischen, Lebensmittel- und Tabakindustrie Anwendung finden.

Stamm Brevibacterium flavum AA6, registriert unter Nr. B-3992 am 01. April 1987. .

5. Verfahren nach Anspruch 1, dadurch g e k e n n - z e i c h n e t , daß man die Mutanten der Mikroorganismen der Art Corynebacterium glutamicum verwendet, die im Allunionsofrschungsinstitut für Genetik und Selektion von industriemä igen Mikroorganismen (VNIIGenetika) deponiert sind:

Stamm Corynebacterium glutamicum AA41, registriert unter Nr. B-3321 am 25. März 1985,

Stamm Corynebacterium glutamicum AA11, registriert unter Nr. B-3323 am 25. März 1985.

6. (geändert) Biologisch reine Kulturen der Mikroorganismen Kennlinien aufweisen und mit einem beliebigen der nachstehend ausgeführten Stämme - Produzenten von L-Alanin, identifiziert werden: ·

Brevibacterium flavum AA1, registriert unter Nr. B-3061 am 05. April 1984;

Brevibacterium flavum AA2, gregistriert unter Nr. B-3062 am 05. April 1984;

Brevibacterium flavum AA5, registriert unter Nr. B-3991 am 01. April 1987;

Brevibacterium flavum AA6, registriert unter Nr. B-3992 am 01. April 1987;

Corynebacterium glutamicum AA41, registriert unter Nr. B-3321 am 25. März 1985;

Corynebacterium glutamicum AA11, registriert unter Nr. B-3323 am 25. März 1985.

# INTERNATIONAL SEARCH REPORT

International Application No PCT/SU89/00111

## I. CLASSIFICATION OF SUBJECT MATTER (if several classification symbols apply, indicate all) 6

According to International Patent Classification (IPC) or to both National Classification and IPC

IPC 4 : C12P 13/06//C12N 15/01 (C12P 11/06, C12R 1:13) (C12P 13/06

## II. FIELDS SEARCHED

C12R 1:15)

### Minimum Documentation Searched 7

| Classification System | Classification Symbols |
|---|---|
| IPC 4 | C 12 P 13/06, C 12 N 15/01 (C 12 P 13/06, C 12R1:13) (C 12 P 13/06, C 12 R1:15) |

### Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | SU, A1, 182646, (T.N LAFITSKAYA et al.) 09 August 1966 (09.08.66) | 1-6 |
| A | US, A, 3898128 (TANABE SEIYAKU CO., Ltd) 05 August 1975 (05.08.75) see the claims | 1-6 |
| A | CH, A5, 651066, (W.R. GRACE & CO), 30 August 1985 (30.08.85), see the claims & GB, B, 2084155, 11.01.84  SE, A, 8104942, 18.03.82  FR, B1, 2490219, 18.04.86  NL, A, 8102156, 16.04.82  JP, A2, 57-83289, 25.05.82  DE, A1, 3134892, 27.05.82  CA, A1, 1178909, 04.12.84 | 1-6 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 14 July 1989 (14.07.89) | 7 August 1989 (07.08.89) |

| International Searching Authority | Signature of Authorized Officer |
|---|---|
| ISA/SU | |

Form PCT/ISA/210 (second sheet) (January 1985)